# EUROPEAN PATENT SPECIFICATION

(11) **EP 1 828 105 B1**
(45) Date of publication and mention of the grant of the patent: **03.09.2008**
(21) Application number: 04821403.5
(22) Date of filing: 22.12.2004
(51) Int. Cl.: C07C 231/12, C07C 231/18, C07C 211/27

(54) **A PROCESS FOR THE PREPARATION OF R-(-)-N, ALPHA-DIMETHYLPHENETHYLAMINE (LEVMETAMFETAMINE) OR S-(+)-N, ALPHA-DIMETHYLPHENETHYLAMINE (METHAMPHETAMINE) FROM d-EPHEDRINE OR L-EPHEDRINE RESPECTIVELY**
VERFAHREN ZUR HERSTELLUNG VON R-(-)-N, ALPHA-DIMETHYLPHENETHYLAMIN (LEVMETAMPHETAMIN) ODER S-(+)-N, ALPHA-DIMETHYLPHENETHYLAMIN (METAMPHETAMIN) AUS D-EPHEDRIN BZW. L-EPHEDRIN
PROCEDE POUR LA PREPARATION DE LA R-(-)-N, ALPHA-DIMETHYLPHENETHYLAMINE (LEVMETAMFETAMINE) OU DE LA S-(+)-N, ALPHA-DIMETHYLPHENETHYLAMINE (METHAMPHETAMINE), A PARTIR DE LA D-EPHEDRINE OU L-EPHEDRINE RESPECTIVEMENT

(43) Date of publication of application: 05.09.2007
(73) Proprietor: Emmellen Biotech Pharmaceuticals Limited, Powai, Mumbai 400 076, MAH (IN)
(72) Inventor: GOLLAPUDY, Subrahmanyam, Hyderabad 500033 (IN); NITTALLA, Venkata Ramana, Mahad 40209 (IN)
(74) Representative: O'Neill, Michelle
(86) International application number: PCT/IN2004/000398
(87) International publication number: WO 2006/067794

(56) References cited:
- US-B1- 6 399 828
- S. BRUCE KING AND K. BARRY SHARPLESS: "An Efficient Synthesis of Enantiomerically Pure trans-2-Phenylcyclohexanol" TETRAHEDRON LETTERS, vol. 35, no. 31, 1994, pages 5611-5612, XP002346341 cited in the application
- LLEWELLYN H. WELSH: "The constitution of Acetylephedrine and Acetly-psi-ephedrine" J. AM. CHEM. SOC., vol. 69, 1947, pages 128-136, XP002244101

## Description

The present invention relates to an efficient process for the preparation of optically active R-(-)-N, α -Dimethylphenethylamine (Levmetamfetamine) or S-(+)- N, α - Dimethylphenethylamine (Methamphetamine) free of its optical antipode from d-ephedrine or I-ephedrine respectively.

### Background and Prior Art:

The invention relates to a novel process for the synthesis of levmetamfetamine or methamphetamine from d-ephedrine or I-ephedrine. Levmetamfetamine and methamphetamine are the intermediates for the production of Selegiline hydrochloride and Benzaphetamine hydrochloride.

Many methods are known in the literature for making these molecules. The various methods of synthesis of these compounds are categorized broadly in the following types:
- Chiral synthesis
- Racemic synthesis and separation by resolution
- From appropriate ephedrines via chlorination and catalytic hydrogenation
- From appropriate ephedrines via catalytic hydrogenation
- From appropriate ephedrine hydrochlorides via O-acylation and catalytic hydrogenation
   **1**) **a**.) Chiral synthesis of 1-alkyl- 2-phenyl ethylamines via grignard reaction of **4-**phenyl-1,3-oxazolidines and subsequent hydrogenolysis yields levmetamfetamine (Tahkasahi Hiroshi et. al., Chem. Pharm. Bull. 1985,33(11), 4662-70).
      **b**) Other chiral synthesis is from chiral Oxazolidines (Henri Phillipe Husson et. al., Synthetic communications, 1987, 17(6), 669-676). Thus, successive dialkylation of 1,3-Oxazolidines afforded substituted N-cyano methyl-1,3-Oxazolidine which was decyanated using NaBH₄ in ethanol at room temperature and the chiral appendage of this product was removed by hydrogenolysis giving methamphetamine in 56-62% yield. This method though useful to prepare optically pure methamphetamine, is expensive and labor intensive.
   **2**) In the synthesis of racemic methyl amphetamine, various methods were described in the prior art. Some of them are:
      **a**) Racemic Methyl amphetamines were prepared by reductive alkylation hydrogenolysis of phenyl-2-propanone with N-Benzyl methyl amine (Skinner, Harry P., Forensic Sci. Int. 1993, 60 (3) 155-62).
      **b**) The commercial use of the Wallach- Leucart reaction was explored in the synthesis of methyl amphetamines from phenyl acetone and ammonium formate and hydrolysis of the intermediate yielded amphetamines. This was treated with formic acetic anhydride to form (±)- N- formyl amphetamine which was reduced by LAH to get racemic methyl amphetamine in 67 % yield (Cervinka, Otakar et. al., Collect. Czech. Chem. Comm1968, 33 (11) 3551-7).
      c) In the illegal synthesis of methyl amphetamines (Masano Isutsumi et.al., Science and Crime detection (Japan), 1953, 6,50-2), phenyl acetic acid was treated with Pb (OAc)₂ to get phenyl acetone . This was reacted with MeNH₂ and reduced to get racemic methyl amphetamine.
      d) Racemic methyl amphetamine was prepared from acetaldehyde (Adolph C. J. Opfermann et.al.,Brit. 782,887) via grignard reaction with benzyl magnesium chloride in ether and the intermediate was treated with methyl amine.

      The racemic methyl amphetamine obtained via any of these processes is then resolved to get levmetamfetamine and methamphetamine via resolution with optically active carboxylic acids through a lengthy and labour intensive process with less over all yields.
      Hence it is desirable to have the simple and economically viable process to get an optically pure form of these methyl amphetamines.
   **3**) In another procedure, heating the appropriate hydrochloride salts of ephedrine with thionyl chloride at reflux temperature to get the appropriate 1-phenyl-1-chloro-2 methylamino propane hydrochloride, followed by catalytic hydrogenation gave appropriate enantiomers of methyl amphetamines (Hajicek Josef et.al., Czech. CS 272,434). Though the reaction gives appropriate methyl amphetamines, the reaction scheme is undesirable because of the usage of hazardous and eco un-friendly thionyl chloride and expensive palladium based hydrogenating catalysts.
   **4**. **a**) In another procedure, appropriate hydrochloride salts of Ephedrine were converted directly to appropriate methyl amphetamines by hydrogenating in glacial acetic acid using sulphuric acid and Pd wool (Karl Kindler et. al., Ann., 1948, 560,215-21).
      **b**) In another procedure, appropriate hydrochloride salts of Ephedrine were converted directly to appropriate methyl amphetamines by hydrogenating in glacial acetic acid using hydrochloric acid and Pd at 90°C (Knoll et.al., Ger. 968,545).
      **c**) In another procedure, 1-ephedrine was reacted with Phosphorous oxychloride to give 1-2 -Chloro-3,4-dimethyl -5-phenyl-1,3,2-oxaazaphospholidine-2-oxide . Hydrolysis of this yielded 1-ephedrine Phosphate, which was catalytically hydrogenated to form the methamphetamine (A.Larizza et. al., J. Med. Chem.,1966, 9, 966 -67).
      **d**) One of the illegal manufactures of methyl amphetamines is from appropriate ephedrines via reduction with hydroiodic acid and red phosphorous (Skinner, Harry F et. al., Forensic Sci. Int. 1990, 48 (2),123-34). This method has disadvantages which includes hydroiodic acid is toxic and strong irritant and contact must be minimised. Red phosphorous is a flammable and explosive solid and must be handled with care. So another route was desirable which could overcome these problems.
   **5**) Recent process (Robert Fredrick Rosewell et. al , US 6,399,828) for the preparation of Methyl amphetamine involves O-acylation of the appropriate hydrochloride salts of ephedrine to hydrochloride salts of O-Acetyl ephedrine and subsequent hydrogenolysis provides methyl amphetamines. Thus methamphetamine was obtained from either 1R, 2S-(-) ephedrine or 1S,2S-(+)-pseudoephedrine via O-acylation and hydrogenolysis process. Though this process produces required methyl amphetamines, it is an expensive process because of the cost of the hydrogenating catalyst, which is Palladium on carbon (Pd/C).

Thus, there was a long felt need for a process which is eco-friendly, cost effective and less labor intensive. The process of the present invention was investigated on these lines and found to be good in all respects. Using the present invention levmetamfetamine and methamphetamine were prepared in high overall yields from d-ephedrine and 1-ephedrine respectively in an efficient, cost effective and industrially safe manner as will be evident from the accompanying detailed description.

### Objects of the invention:

It is thus an object of the invention to provide a process for the preparation of R-(-)-N, α -Dimethylphenethylamine (Levmetamfetamine) or S-(+)-N, α-Dimethylphenethylamine (Methamphetamine) from d-ephedrine or 1-ephedrine respectively, that overcomes the drawbacks existing in the prior art.

It is a further object of the invention to provide a process for preparation of optically active R-(-)-N, α -Dimethylphenethylamine (Levmetamfetamine) or S-(+)-N, α - Dimethylphenethylamine (Methamphetamine) from d-ephedrine or 1-ephedrine respectively, free of its optical antipode.

Yet another object of the invention is to provide a process for the preparation of optically active R-(-)-N, α -Dimethylphenethylamine (Levmetamfetamine) or S-(+)- N, α - Dimethylphenethylamine (Methamphetamine) from d-ephedrine or 1-ephedrine respectively, that has a high overall yield of R-(-)-N, α -Dimethylphenethylamine (Levmetamfetamine) or S-(+)-N, α - Dimethylphenethylamine (Methamphetamine).

A further object of the invention is to provide a process for the preparation of R-(-)-N, α -Dimethylphenethylamine (Levmetamfetamine) or S-(+)-N, α - Dimethylphenethylamine (Methamphetamine) from d-ephedrine or 1-ephedrine respectively, that is cost effective and industrially feasible.

Yet another object of the invention is to provide a process for the preparation of R-(-)-N, α -Dimethylphenethylamine (Levmetamfetamine) or S-(+)-N, α - Dimethylphenethylamine (Methamphetamine) from d-ephedrine or 1-ephedrine respectively, that avoids use of complex and cost extensive reagents and precious metal hydrogenating catalysts.

### Summary of the invention.

The present invention relates to a process for asymmetric synthesis of R-(-)-N, α - Dimethylphenethylamine (Levmetamfetamine) of formula I 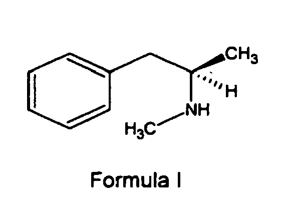 from d-ephedrine precursor or S-(+)-N, α - Dimethylphenethylamine (Methamphetamine) enantiomer of formula II, 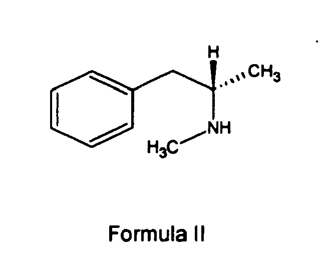 from 1-ephedrine precursor, which are free from their optical antipode comprising:
a) Reacting d-ephedrine or 1-ephedrine base of formula III 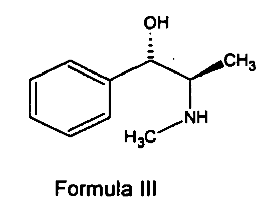 or Formula IV 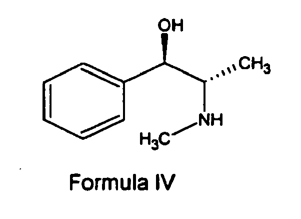 with an acylating agent to make a reaction mixture containing a N- acylated ephedrine of formula V 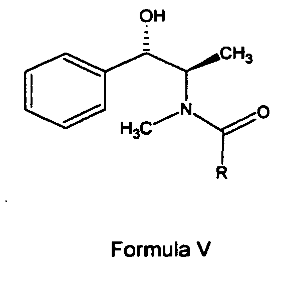 or Formula VI, 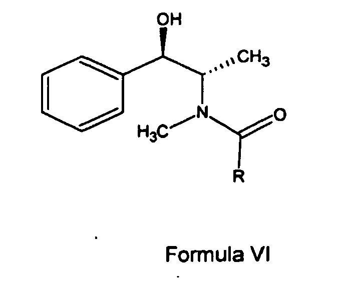
b) deoxygenation of N-acylated ephedrines ( formula V or VI) to make the compound of the formula VII 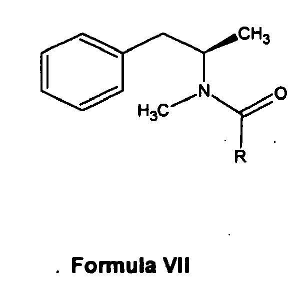 or Formula VIII 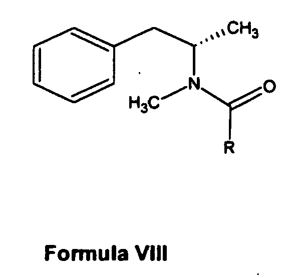 by using Raney Nickel catalyst.
c) effecting acid hydrolysis of the above deoxygenated products to get the compound of formula I or II.

### Detailed description:

After considerable investigation of the prior art and various modifications thereof, it was concluded that a new approach was required for development of cost effective process for the preparation of R-(-)-N, α -Dimethylphenethylamine (Levmetamfetamine) or S-(+)-N, a - Dimethylphenethylamine (Methamphetamine). Further, the literature procedures for making amphetamines and methamphetamines report the retention of configuration at the carbon bearing amino group (.Noggle, Deruiter, and Clarke, J. Chrom. Sci., 1987, 25, 38-42); Allen and Kiser, J. Forensic Sciences., 1987 , 32,(4) ,953-962). Therefore d,1 ephedrine is expected to give d,1-methyl amphetamines , d-ephedrine is expected to give levmetamfetamine and 1-ephedrine is expected to give methamphetamine respectively. Thus, the obvious choice of starting materials were d-ephedrine or 1-ephedrine since they have the right stereochemistry at C-2 carbon atom of the molecule. These compounds are further advantageous in that they are easily available commercially.

The process of the present invention comprises (a) amide formation and (b) removal of the benzylic hydroxyl group by refluxing with Raney Nickel catalyst and (c) subsequent hydrolysis of the amide group. When this process is applied to the production of levmetamfetamine and methamphetamine, the process has several advantages over current production methods, which are:
- Shorter cycle times
- Convenient work up (filtration and solvent removal)
- Economic viability because of the use of inexpensive and easily available catalyst
- No additional hydrogenator required in the reaction route, and
- Better chemical hygiene and eco-friendliness
- Avoids the use of metal hydrides, which are not suitable for plant scale operations.
- Use of industrially safe reagents.

Further optimization of yields and operation cycle times using optimization methods known to those skilled in the art would only increase these advantages.

The present inventors have surprisingly found that Raney Nickel catalyst can be used for deoxygenation of benzylic alcoholic group. This catalyst is hitherto unreported for the deoxygenation of the secondary benzylic alcohols. It was in fact known that Raney Nickel catalyst deoxygenates tertiary alcohols to the corresponding hydrocarbons (J. Amer. Chem. Soc., 1967 ,89, 4233 ; Tet. Lett.., 1994, 35,5611 ; J. Org.Chem., 1988 , 53, 432 and 3158). The use of Raney Nickel catalyst for deoxygenation of benzylic alcohol is not only cost effective since Raney Nickel is inexpensive compared to other precious catalysts such as Pd-C, Pd-BaSO₄ and Pt-O₂ which are normally used for deoxygenations but also industrially safe. One of the other advantages of the present invention is the convenient workup of every step including Raney Nickel reaction-filtration and solvent removal. This synthetic route for the deoxygenation of secondary benzylic alcohol is applicable to related compounds as well, with substitution patterns obvious to those skilled in the art and are intended to be within the scope of the present invention.

The general reaction scheme employed in the present invention is as shown in the accompanying figure:

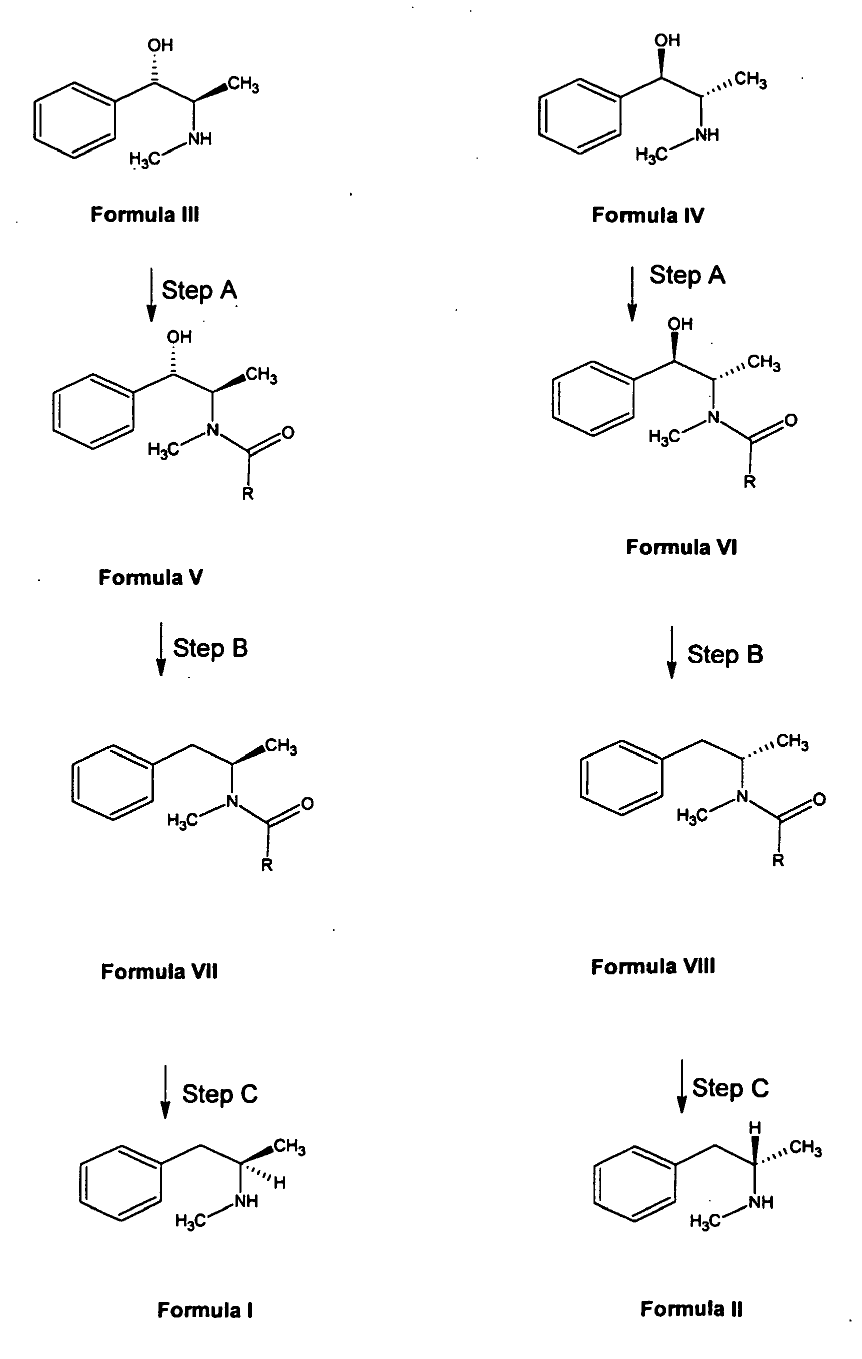

In step A, the starting material d-ephedrine of formula III or 1-ephedrine of formula IV are converted into their corresponding amides of formula V or formula VI, wherein R may be hydrogen, methyl or phenyl group using an appropriate reagent. When R is hydrogen, the appropriate reagent is ethyl formate. When R is methyl group, the appropriate reagent is acetic anhydride and when R is phenyl group, the appropriate reagent is benzoyl chloride. In the most preferred embodiment of the invention, R corresponds to hydrogen atom wherein the appropriate reagent used is ethyl formate.

In step B, the corresponding amides of formula V or formula VI are deoxygenated using Raney Nickel as reagent in refluxing toluene to obtain the corresponding deoxygenated amides of formula VII or VIII wherein R may be as defined above.

In step C, the deoxygenated amides of formula VII or VIII are hydrolysed by refluxing in 1:1 aq. HCl to the corresponding title compounds of the formula I (Levmetamphetamine) or formula II (Methamphetamine) in surprisingly good yields.

The present invention thus provides an efficient process for stereoselective preparation of optically active R-(-)-N, α -Dimethylphenethylamine (Levmetamfetamine) or S-(+)-N, α Dimethylphenethylamine (Methamphetamine) from d-ephedrine or 1-ephedrine respectively, free from its optical antipode.

An important feature of the present invention is the deoxygenation of N-acyl ephedrine by means of an easily accessible and inexpensive Raney Nickel catalyst. The first step (step A) in the reaction scheme is amide formation of ephedrine. N-Formyl ephedrines of Formula V(a) or VI(a)( when R is hydrogen) were prepared by known process from appropriate ephedrine and ethyl formate (Dieter Enders et. al., Liebigs Ann / Recueil, 1997, 1101-1113).

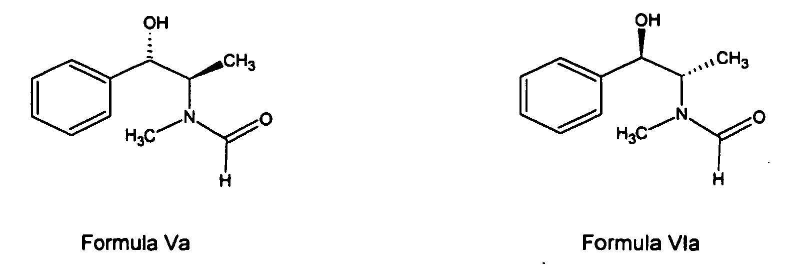

However these compounds can be prepared by methyl formate also. The temperature of reaction is between 50°C and 60°C, preferably between 55°C and 60°C. The reaction is carried out by mixing appropriate ephedrine and ethyl formate and carefully heating the reaction mixture to 55°C-60°C and the product was isolated by distilling off the excess reagent and ethanol.

N-Acetyl ephedrines [Formula V (b) and Formula VI (b)] is prepared from appropriate ephedrine and acetic anhydride in chloroform.

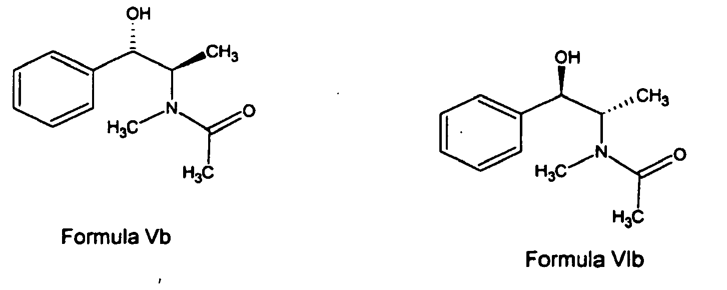

N-Benzoyl ephedrines [Formula V (c) and Formula VI (c)] is prepared from appropriate ephedrine and benzoyl chloride in an organic solvent using aq. sodium hydroxide at 20°C temperature.

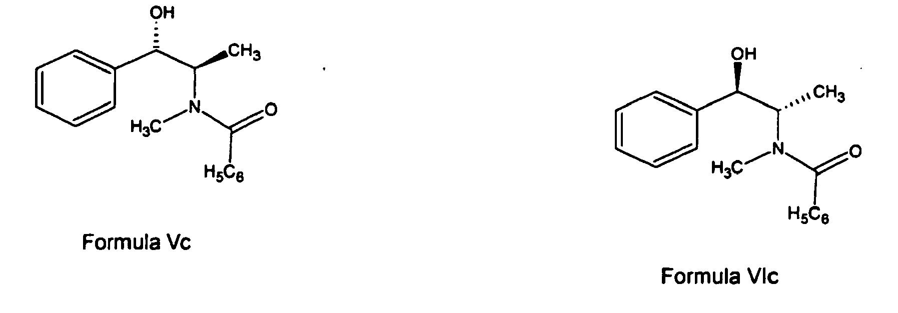

The organic solvent for this purpose may be any suitable solvent known to a person skilled in the art. The solvent may be preferably selected from dichloromethane or chloroform.

The second step (step B), which is a key reaction in the reaction scheme is benzylic secondary alcohol deoxygenation of the above ephedrine amides. The reaction is carried out by mixing appropriate ephedrine amides (N-formyl, N-acetyl, or N-benzoyl) and Raney Nickel catalyst in a suitable organic solvent and slowly heating the reaction mixture to reflux temperature to obtain compounds of Formula VII(a), VII(b) or VII(c) or VIII(a), VIII(b) or VIII(c).

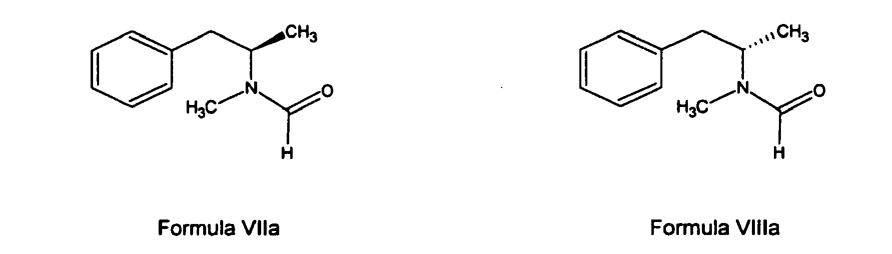

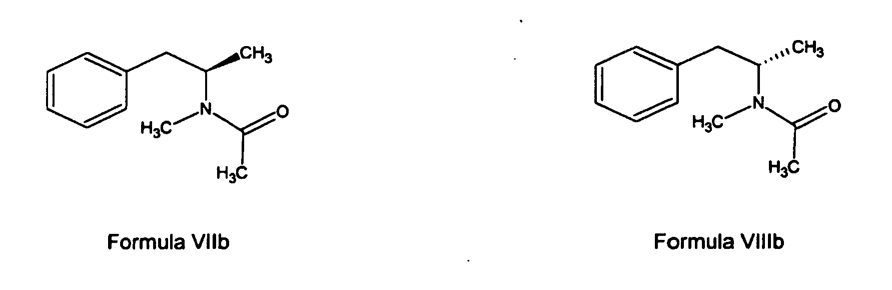

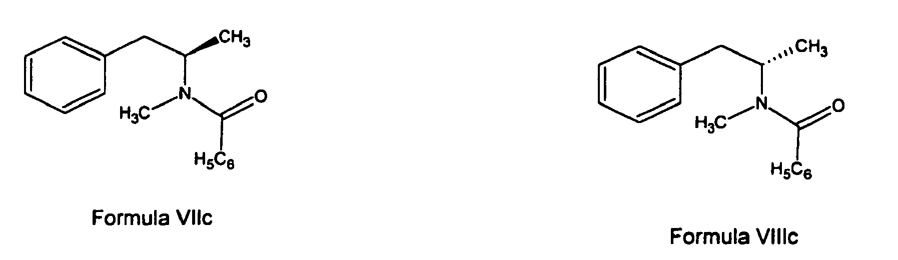

The organic solvent for this reaction may preferably be selected from toluene or benzene. Most preferably the solvent is toluene. The ratio of the ephedrine amide and Raney Nickel catalyst is crucial for this reaction. The ratio of ephedrine amide and Raney Nickel catalyst could be any thing between one gram to six ml. (settled catalyst in water) and one gram to two ml. Preferably the ratio is one gram to two ml. There is no appreciable deoxygenation of ephedrine amides below this range.

The temperature of reaction mixture for the deoxygenation is maintained between 70°C and 85° C. The optimum temperature is in between 80°C and 85°C. The product is recovered by filtering the catalyst and solvent evaporation. This crude product is reasonably pure enough to carry out hydrolysis without further purification and it contains mainly deoxygenated ephedrine amide. For all practical purpose N- acyl-ephedrine compounds were deoxygenated in high yield and these examples are described in the following section.

The third step (step C) in the scheme is hydrolysis of desoxy ephedrine amides. This step was carried out by mixing the appropriate desoxy ephedrine amide and 1:1 aq. HCl in the ratio of 1 gm to 5 ml. and then heating to reflux temperatures. The optimum temperature for this reaction is in between 103°C and 108°C

The reaction mixture is extracted with a non-polar organic solvent (preferably toluene) to remove non basic impurities and the the aqueous layer was basified with aqueous sodium hydroxide. The product was taken into solvent and isolated the product by solvent removal. The solvent used for this extraction may preferably selected from a group comprising of benzene, toluene, methylene dichloride, diethyl ether and diisopropyl ether or mixtures thereof

The details of the invention, its objects and advantages are explained hereunder in greater detail in relation to non-limiting exemplary illustrations. The examples are merely illustrative and do not limit the teaching of this invention and it would be obvious that various modifications or changes in the procedural steps by those skilled in the art without departing from the scope of the invention and shall be consequently encompassed with in the ambit and spirit of this approach and scope thereof.

In the following experiments the analytical instruments used for qualitative analysis includes Gas liquid chromatography, Infrared spectrometer and Polarimeter and thin layer chromatography. d- and 1-Ephedrine bases are commercially available and were used to illustrate in the reactions useful in the process.

### Example 1: Preparation of levmetamfetamine:

### Step A: Preparation of N-Formyl ephedrine (V a) from d-ephedrine (III).

In a three-necked 500 ml round bottom flask equipped with thermo controller, stirrer and condenser was charged with 150 grams (0.9mole) of d-ephedrine and ethyl formate 115 grams (1.55 mole). The reaction mixture was warmed with a water bath to 55-60°C under stirring and maintained at this temperature for eight hours. Distilled off the excess reagent and formed ethanol. N-Formyl derivative was obtained as pale yellow to white viscous liquid (170 grams) Specific Optical rotation: - 22.10° (2% solution in methanol), purity: (GLC: 99.6%)

### Step B: Preparation of N-Formyl desoxy ephedrine (VIIa) from N-Formyl Ephedrine (Va)

In a three necked 2 Lit. round bottom flask equipped with thermo controller, stirrer and condenser was charged with Raney Nickel catalyst (slurry weight 345 geams; slurry volume 190 ml.) and 100 grams (0.51 mole) of N-Formyl ephedrine and distlled toluene (0.8 lit.). The reaction mixture was heated on a water bath to reflux under stirring for fifteen hours. Cooled the reaction mixture to 60°C and filtered the catalyst and washed the catalyst with toluene (500 ml) twice. The combined toluene layers were washed with water and dried over sodium sulphate and concentrated under vacuum which afforded N formyl desoxyephedrine as a pale yellow to white clear viscous liquid toluene (73 grams, 80% yield).

### Step C: Preparation of levmetamfetamine (I) from N-Formyl desoxy ephedrine (VIIa)

In a three necked 500 ml. round bottom flask equipped with thermo controller, stirrer and condenser was charged with N-formyl desoxy ephedrine (50 grams, 0.28 mole) and 1:1 aq. HCl (250 ml). The reaction mixture was refluxed (reaction temperature 104 - 105°C) while stirring for 10 hours. Cooled the reaction mass to 40°C and extracted with toluene to remove neutral impurities. Aqueous layer was basified with aq. Sodium hydroxide and extracted with diisopropyl ether (2 X 250 ml). The organic layers were washed with water and dried over sodium sulphate. Solvent was removed by distillation under vacuum, which afforded levmetamfetamine as a pale yellow to white clear liquid. (38 grams, 90 % yield), Specific optical rotation.: -17.4° (2% solution in 1.2N HCl); purity: (GLC: 98.5%)

### Example 2: Preparation of methamphetamine

### Step A: Preparation of N-Formyl ephedrine (VIa) from 1-ephedrine (IV).

In a three-necked 500 ml round bottom flask equipped with thermo controller, stirrer and condenser was charged with 200 grams (1.2 mole) of 1-ephedrine and ethyl formate 160 grams (2.16 mole). The reaction mixture was warmed with a water bath to 55-60°C under stirring and maintained at this temperature for eight hours. Distilled off the excess reagent and formed ethanol. N-Formyl derivative was obtained as pale yellow to white viscous liquid (220 grams), Specific optical rotation: +22.90° (2% solution in methanol), purity: (GLC): 99.90%

### Step B : Preparation of N-Formyl desoxy ephedrine (VIIIa) from N-Formyl Ephedrine (VIa)

In a three necked 1lit. round bottom flask equipped with thermo controller, stirrer and condenser was charged with Raney Nickel catalyst (slurry weight 180 grams; slurry volume 98 ml.) and 50 grams (0.26mole) of N-Formyl ephedrine and distilled toluene (0.2 lit.). The reaction mixture was heated on a water bath to reflux (reaction temperature 84°C) under stirring for fifteen hours. Cooled the reaction mixture to 60°C and filtered the catalyst and washed the catalyst with toluene (200 ml) twice. The combined toluene layers were washed with water and dried over sodium sulphate and concentrated under vacuum which afforded N-formyl desoxyephedrine as a pale yellow to white clear viscous liquid (36 grams, 80% yield).

### Step C: Preparation of methamphetamine (II) from N-Formyl desoxy ephedrine (VIIIa)

In a three necked 500 ml. round bottom flask equipped with thermo controller, stirrer and condenser was charged with N-formyl desoxy ephedrine (35 grams, 0.197 mole) and 1:1 aq. HCl (175 ml). The reaction mixture was refluxed (reaction temperature 104-105°C) while stirring for 10 hours. Cooled the reaction mass to 40°C and extracted with toluene to remove neutral impurities. Aqueous layer was basified with aq. Sodium hydroxide and extracted with diisopropyl ether (2 X 100 ml). The organic layers were washed with water and dried over sodium sulphate. Solvent was removed by distillation under vacuum, which afforded methamphetamine as a pale yellow to white clear liquid. (23 grams, 78 % yield), Specific optical rotation.: + 17.3° (2% solution in 1.2N HCl) ; purity: (GLC): 98.2%].

### Example 3: Preparation of levmetamfetamine:

### Step A: Preparation of N-Acetyl ephedrine (V b) from d-ephedrine (III).

In a three-necked 1000 ml round bottom flask equipped with thermo controller, stirrer and condenser was charged with 100 grams (0.606 mole) of d-ephedrine and acetic anhydride 185.5 grams (1.818 mole). The reaction mixture was warmed with a water bath to 65-70°C under stirring and maintained at this temperature for two hours. Added 250 ml water. Extracted the N-acetyl derivative with toluene(3x150 ml). The combined organic layer was concentrated to get N-acetyl derivative (113 grams,MP:85-88°C).

### Step B: Preparation of N-Acetyl desoxy ephedrine (VIIb) from N-Acetyl Ephedrine (Vb)

In a three necked 2 Lit. round bottom flask equipped with thermo controller, stirrer and condenser was charged with Raney Nickel catalyst (slurry weight 345 grams; slurry volume 190 ml.) and 100 grams (0.483 mole) of N-acetyl ephedrine in distlled toluene (0.8 lit.). The reaction mixture was heated on a water bath to reflux under stirring for fifteen hours. Cooled the reaction mixture to 60°C and filtered the catalyst and washed the catalyst with toluene (500 ml) twice. The combined toluene layer was washed with water and dried over sodium sulphate and concentrated under vacuum which afforded N Acetyl desoxyephedrine as a pale yellow to white clear viscous liquid (64 grams, 69.41 % yield).

### Step C: Preparation of levmetamfetamine (I) from N-Acetyl desoxy ephedrine (VIIb)

In a three necked 500 ml. round bottom flask equipped with thermo controller, stirrer and condenser was charged with N-acetyl desoxy ephedrine (50 grams, 0.261 mole) and 1:1 aq. HCl (250 ml). The reaction mixture was refluxed (reaction temperature 104 - 105°C) while stirring for 12 hours. Cooled the reaction mass to 40°C and extracted with toluene to remove neutral impurities. Aqueous layer was basified with aq. Sodium hydroxide and extracted with diisopropyl ether (2 X 200 ml). The organic layers were pooled and washed with water and dried over sodium sulphate. Solvent was removed by distillation under vacuum, which afforded levmetamfetamine as a pale yellow to white clear liquid. (35 grams, 89.7% yield), Specific optical rotation.: -17.6° (2% solution in 1.2N HCl); purity : (GLC: 98.9%)

### Example 4: Preparation of methamphetamine

### Step A: Preparation of N-Acetyl ephedrine (VIb) from 1-ephedrine (IV).

In a three-necked 1000 ml round bottom flask equipped with thermo controller, stirrer and condenser was charged with 100 grams (0.606 mole) of 1-ephedrine and acetic anhydride 185.5 grams (1.818 mole). The reaction mixture was warmed with a water bath to 65-70°C under stirring and maintained at this temperature for two hours. Added 250 ml water. Extracted the N-acetyl derivative with toluene(3x150 ml). The combined organic layer was concentrated to get N-acetyl derivative (110 grams, MP: 86-87°C)

### Step B : Preparation of N-Acetyl desoxy ephedrine (VIIIb) from N-Acetyl Ephedrine (VIb)

In a three necked 2 Lit. round bottom flask equipped with thermo controller, stirrer and condenser was charged with Raney Nickel catalyst (slurry weight 345 grams; slurry volume 190 ml.) and 100 grams (0.483 mole) of N-acetyl ephedrine(VIb) in distlled toluene (0.8 lit.). The reaction mixture was heated on a water bath to reflux under stirring for fifteen hours. Cooled the reaction mixture to 60°C and filtered the catalyst and washed the catalyst with toluene (500 ml) twice. The combined toluene layer was washed with water and dried over sodium sulphate and concentrated under vacuum which afforded N Acetyl desoxyephedrine as a pale yellow to white clear viscous liquid (66 grams, 71.70% yield).

### Step C: Preparation of methamphetamine (II) from N-Acetyl desoxy ephedrine (VIIIb)

In a three necked 500 ml. round bottom flask equipped with thermo controller, stirrer and condenser was charged with N-acetyl desoxy ephedrine (50 grams, 0.261 mole) and 1:1 aq. HCl (250 ml). The reaction mixture was refluxed (reaction temperature 104 - 105°C) while stirring for 12 hours. Cooled the reaction mass to 40°C and extracted with toluene to remove neutral impurities. Aqueous layer was basified with aq. Sodium hydroxide and extracted with diisopropyl ether (2 X 200 ml). The organic layers were pooled and washed with water and dried over sodium sulphate. Solvent was removed by distillation under vacuum, which afforded methamphetamine as a pale yellow to white clear liquid. (34 grams, 87.17% yield), Specific optical rotation.: +17.8° (2% solution in 1.2N HCl); purity: (GLC: 99%)

### Example 5: Preparation of levmetamfetamine:

### Step A: Preparation of N-Benzoyl ephedrine (V c) from d-ephedrine (III).

In a three-necked 1000 ml round bottom flask equipped with thermo controller, stirrer and condenser was charged with 100 grams (0.606mole) of d-ephedrine in chloroform (175 ml). Added benzoyl chloride (91.99 grams) and 20% sodium hydroxide solution ( 134 ml) simultaneously at room temperature. The reaction mass was stirred further 90 minutes at room temperature. Seperated the organic layer and aq. layer .Extracted the aq. layer with another 175 ml of chloroform. The organic layers were pooled and washed with water and concentrated. The residue was recrystallised in benzene/pet. ether which gave the product (128 grams, 78.45%)

### Step B: Preparation of N-Benzoyl desoxy ephedrine (VIIc) from N-Benzoyl Ephedrine (Vc)

In a three necked 2 Lit. round bottom flask equipped with thermo controller, stirrer and condenser was charged with Raney Nickel catalyst (slurry weight 350 grams; slurry volume 193 ml.) and 100 grams (0.37 mole) of N-benzoyl ephedrine and distlled toluene (1 lit.). The reaction mixture was heated on a water bath to reflux under stirring for fourteen hours. Cooled the reaction mixture to 60°C and filtered the catalyst and washed the catalyst with toluene (500 ml) twice. The combined toluene layers were washed with water and dried over sodium sulphate and concentrated under vacuum which afforded N Benzoyll desoxyephedrine as a pale yellow to white clear viscous liquid toluene (65.4 grams, 69.57% yield).

### Step C: Preparation of levmetamfetamine (I) from N-Benzoyl desoxy ephedrine (VIIc)

In a three necked 500 ml. round bottom flask equipped with thermo controller, stirrer and condenser was charged with N-benzoyl desoxy ephedrine (50 grams, 0.1976 mole) and 1:1 aq. HCl (250 ml). The reaction mixture was refluxed (reaction temperature 104 - 105°C) while stirring for 13 hours. Cooled the reaction mass to 60°C and extracted with chloroform to remove neutral impurities. Aqueous layer was basified with aq. Sodium hydroxide and extracted with diisopropyl ether (2 X 200 ml). The organic layers were pooled and washed with water and dried over sodium sulphate. Solvent was removed by distillation under vacuum, which afforded levmetamfetamine as a pale yellow to white clear liquid. (20.8 grams, 71 % yield), Specific optical rotation.: -17.2° (2% solution in 1.2N HCl); purity : (GLC: 99%)

### Example 6: Preparation of methamphetamine

### Step A: Preparation of N-Benzoyl ephedrine (VIc) from 1-ephedrine (IV).

In a three-necked 1000 ml round bottom flask equipped with thermo controller, stirrer and condenser was charged with 100 grams (0.606mole) of 1-ephedrine in chloroform (175 ml). Added benzoyl chloride (91.99 grams) and 20% sodium hydroxide solution ( 134 ml) simultaneously at room temperature. The reaction mass was stirred further 90 minutes at room temperature. Seperated the organic layer and aq. layer .Extracted the aq. layer with another 175 ml of chloroform. The organic layers were pooled and washed with water and concentrated. The residue was recrystallised in benzene/pet. ether which gave the product (126.5 grams, 78.% yield)

### Step B : Preparation of N-Benzoyl desoxy ephedrine (VIIIc) from N-Benzoyl Ephedrine (VIc)

In a three necked 2 Lit. round bottom flask equipped with thermo controller, stirrer and condenser was charged with Raney Nickel catalyst (slurry weight 348 grams; slurry volume 193 ml.) and 100 grams (0.37 mole) of N-benzoyl ephedrine and distlled toluene (1 lit.). The reaction mixture was heated on a water bath to reflux under stirring for fourteen hours. Cooled the reaction mixture to 60°C and filtered the catalyst and washed the catalyst with toluene (500 ml) twice. The combined toluene layers were washed with water and dried over sodium sulphate and concentrated under vacuum which afforded N Benzoyl desoxyephedrine as a pale yellow to white clear viscous liquid (65.grams, 69.5% yield).

### Step C: Preparation of methamphetamine (II) from N-Benzoyl desoxy ephedrine (VIIIc)

In a three necked 500 ml. round bottom flask equipped with thermo controller, stirrer and condenser was charged with N-benzoyl desoxy ephedrine (50 grams, 0.1976 mole) and 1:1 aq. HCl (250 ml). The reaction mixture was refluxed (reaction temperature 104 - 105°C) while stirring for 13 hours. Cooled the reaction mass to 60°C and extracted with chloroform to remove neutral impurities. Aqueous layer was basified with aq. Sodium hydroxide and extracted with diisopropyl ether (2 X 200 ml). The organic layers were pooled and washed with water and dried over sodium sulphate. Solvent was removed by distillation under vacuum, which afforded methamphetamine as a pale yellow to white clear liquid. (21.0 grams, 71 % yield), Specific optical rotation.: +17.4° (2% solution in 1.2N HCl); purity : (GLC: 98.99%)

## Claims

1. A process for asymmetric synthesis of R-(-)-N, α -Dimethylphenethylamine (Levmetamfetamine) of formula I 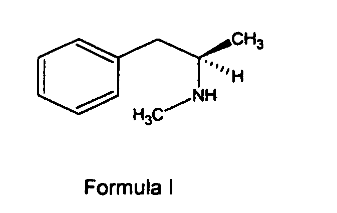 from d-ephedrine precursor, free from its optical antipode comprising:
a) reacting d-ephedrine base of formula III 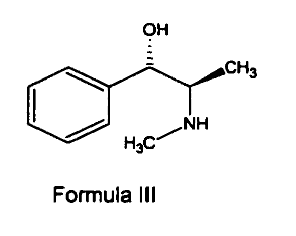 with an acylating agent to make a reaction mixture containing a N- acylated ephedrine derivative of formula V 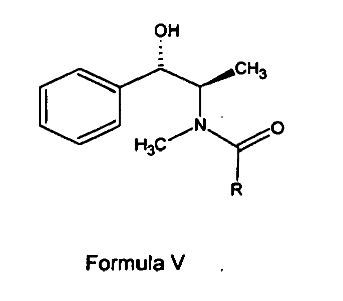 wherein R may be selected from hydrogen, methyl or phenyl group.
b) deoxygenation of N-acylated ephedrines of Formula V to make the compound of the formula VII 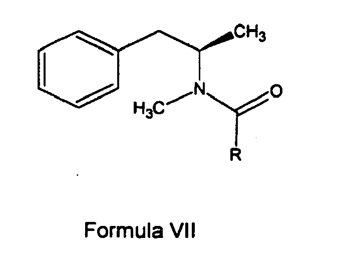 wherein R may be as defined above in step (a).
c) effecting acid hydrolysis of the above deoxygenated products to get the compound of formula I, 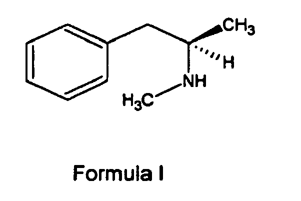
**characterized in that** the deoxygenation reaction in step (b) is carried out in presence of raney nickel catalyst.

2. A process for asymmetric synthesis of S-(+)-N, α - Dimethylphenethylamine (Methamphetamine) enantiomer of formula II, 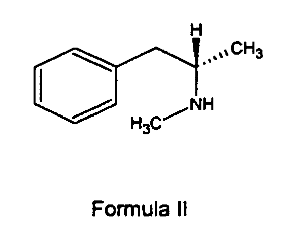 from 1-ephedrine precursor, free from its optical antipode comprising:
a) reacting 1-ephedrine base of formula IV 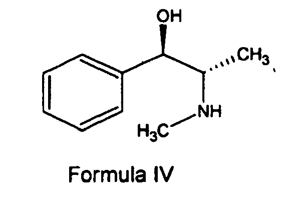 with an acylating agent to make a reaction mixture containing a N- acylated ephedrine of formula VI, 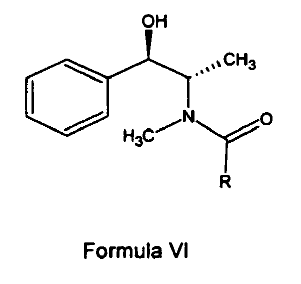 wherein R may be selected from hydrogen, methyl or phenyl group
b) deoxygenation of N-acylated ephedrines of formula VI to make the compound of the formula VIII 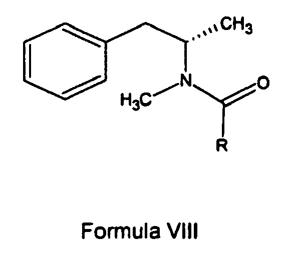 wherein R may be as defined above in step (a).
c) effecting acid hydrolysis of Formula VIII to obtain the compound of formula II, 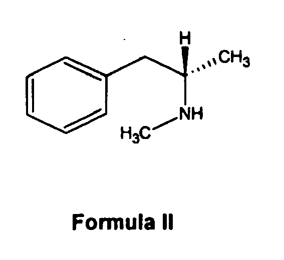
**characterized in that** the deoxygenation reaction in step (b) is carried out in presence of raney nickel catalyst.

3. A process as claimed in claim 1 or 2 wherein, the acylating agent is selected from ethyl formate, methyl formate, acetic anhydride and benzoyl chloride.

4. A process as claimed in claim 3, wherein the acylating agent is ethyl formate.

5. A process as claimed anyone of the preceding claims, wherein the solvent used for deoxygenation is selected from toluene or benzene.

6. A process as claimed in claim 5, wherein the solvent used for deoxygenation is toluene.

7. A process as claimed in anyone of the preceding claims, wherein the temperature used for deoxygenation reaction is the refluxing temperature of the solvent used.

8. A process as claimed in claim 7, wherein the temperature used for the deoxygenation reaction is between 70°C to 85°C.

9. A process as claimed in claim 8, wherein the temperature used for the deoxygenation reaction is between 80°C to 85°C.

10. A process as claimed in anyone of the preceding claims, **characterized in that** the ratio of N-acyl ephedrine (gm) to Raney nickel catalyst (settled volume,ml.) for deoxygenation reaction is 1 gm of substrate per 6 ml of catalyst.

11. A process as claimed in claim 14, wherein the ratio of N-acyl ephedrine (gm) to Raney nickel catalyst (settled volume,ml.) for deoxygenation reaction is 1 gm of substrate per 2 ml of catalyst.

12. A process as claimed in claim 1, 2 or 3, **characterized in that** the acid hydrolysis is carried out using aqueous hydrochloric acid.

## Patentansprüche

1. Verfahren zur asymmetrischen Synthese von R-(-)-N,α-Dimethylphenethylamin (Levmetamfetamin) der Formel I 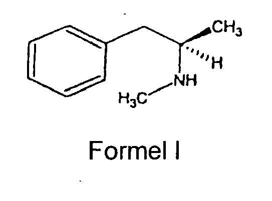 aus d-Ephedrin-Vorläufer, das von seinem optischen Antipoden frei ist, umfassend:
a) man setzt d-Ephedrinbase der Formel III 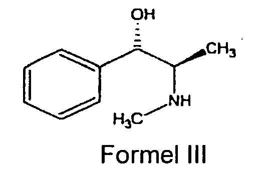 mit einem Acylierungsmittel zu einem Reaktionsgemisch um, das ein N-acyliertes Ephedrinderivat der Formel V enthält, 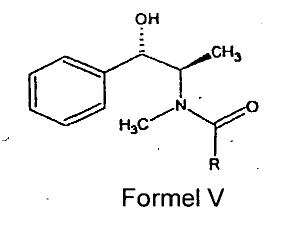 wobei R Wasserstoff, eine Methyl- oder Phenylgruppe ist,
b) man entoxidiert N-acylierte Ephidrine der Formel V zur Bildung der Verbindung der Formel VII 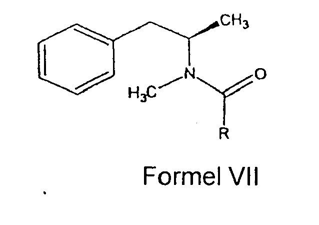 in der R die oben in Stufe a) gegebene Bedeutung haben kann,
c) man führt Säurehydrolyse der oben genannten deoxydierten Produkte zur Erzielung der Verbindung der Formel I durch, 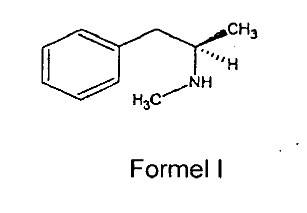
**dadurch gekennzeichnet, dass** die Deoxydierungsreaktion in Stufe b) in Anwesendheit von Raney-Nickel-Katalysator durchgeführt wird.

2. Verfahren zur asymmetrischen Synthese von S-(+)-N,α-Dimethylphenethylamin (Methamphetamin)-Enantiomer der Formel II, 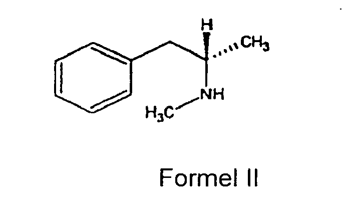 aus 1-Ephedrinvorläufer, der von seinem optischen Antipoden frei ist, umfassend:
a) man setzt 1-Ephedrinbase der Formel IV 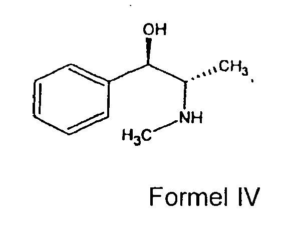 mit einem Acylierungsmittel zu einer Reaktionsmischung, die N-acyliertes Ephedrin der Formel VI enthält, um, 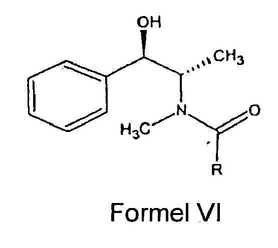 in der R Wasserstoff, eine Methyl- oder Phenylgruppe sein kann,
b) man deoxydiert N-acylierte Ephedrine der Formel VI zur Herstellung der Verbindung der Formel VIII, 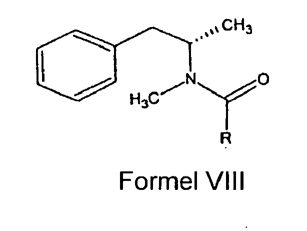 in der R die oben in Stufe a) gegebenen Bedeutung haben kann,
c) man führt Säurehydrolyse der Formel VIII durch zur Erzielung der Verbindung der Formel II, 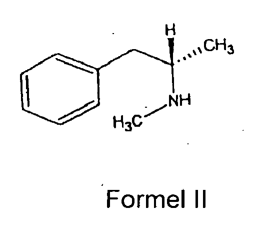
**dadurch gekennzeichnet, dass** die Deoxydierungsreaktion in Stufe b) in Anwesendheit in Raney-Nickel-Kathalysator durchgeführt wird.

3. Verfahren nach Anspruch 1 oder 2, wobei das Acylierungsmittel aus Ethylformiat, Methylformiat, Essigsäureanhydrid oder Benzoylchlorid ausgewählt ist.

4. Verfahren nach Anspruch 3, wobei das Acylierungsmittel Ethylformiat ist.

5. Verfahren nach einem der vorstehenden Ansprüche, wobei das für die Deoxydierung verwendete Lösungsmittel aus Toluol oder Benzol ausgewählt ist.

6. Verfahren nach Anspruch 5, wobei das für die Deoxydierung verwendete Lösungsmittel Toluol ist.

7. Verfahren nach einem der vorstehenden Ansprüche, wobei die für die Deoxydierungsreaktion verwendete Temperatur die Rückflusstemperatur des verwendeten Lösungsmittels ist.

8. Verfahren nach Anspruch 7, wobei die für die Deoxydierungsreaktion verwendete Temperatur zwischen 70°C und 85°C liegt.

9. Verfahren nach Anspruch 8, wobei die für die Deoxydierungsreaktion verwendete Temperatur zwischen 80°C und 85°C liegt.

10. Verfahren nach einem der vorstehenden Ansprüche, **dadurch gekennzeichnet, dass** das Verhältnis von N-Acylephedrin (g) zu Raney-Nickel-Katalysator (abgesetztes Volumen, ml) für die Deoxydierungsreaktion 1 g Substanz auf 6 ml Katalysator ist.

11. Verfahren nach Anspruch 14, wobei das Verhältnis von N-Acylephedrin (g) zu Raney-Nickel-Katalysator (abgesetztes Volumen, ml) für die Deoxydierungsreaktion 1 g Substrat pro 2 ml Katalysator ist.

12. Verfahren nach Anspruch 1, 2 oder 3, **dadurch gekennzeichnet, dass** die Hydrolyse unter Verwendung von wässriger Salzsäure durchgeführt wird.

## Revendications

1. Procédé de synthèse asymétrique de R-(-)-N,α-diméthylphénéthylamine (levmétamfétamine) de formule I 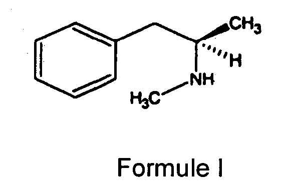 à partir du précurseur d-éphédrine, exempt de son antipode optique, comprenant :
a) la réaction de la base d-éphédrine de formule III 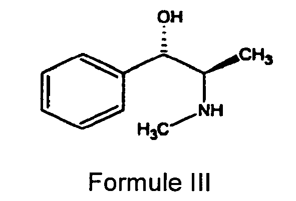 avec un agent d'acylation pour donner un mélange réactionnel contenant un dérivé de N-éphédrine acylée de formule V 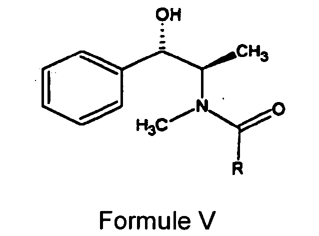 où R peut être choisi parmi un atome d'hydrogène, un groupe méthyle ou un groupe phényle ;
b) la désoxygénation des N-éphédrines acylées de formule V pour donner un produit de formule VII 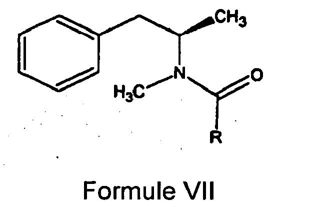 où R peut être tel que défini ci-dessus dans l'étape (a) ;
c) la réalisation d'une hydrolyse acide des produits désoxygénés ci-dessus pour obtenir le composé de formule I 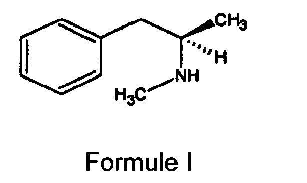
**caractérisé en ce que** la réaction de désoxygénation de l'étape (b) est réalisée en présence d'un catalyseur de nickel de Raney.

2. Procédé de synthèse asymétrique de l'énantiomère S-(+)-N,α-diméthylphénéthylamine (méthamphétamine) de formule II 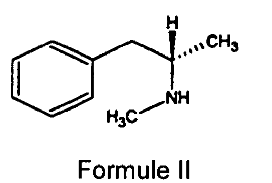 à partir du précurseur 1-éphédrine, exempt de son antipode optique, comprenant :
a) la réaction de la base 1-éphédrine de formule IV 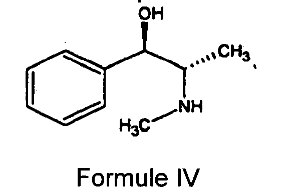 avec un agent d'acylation pour donner un mélange réactionnel contenant un dérivé de N-éphédrine acylée de formule VI 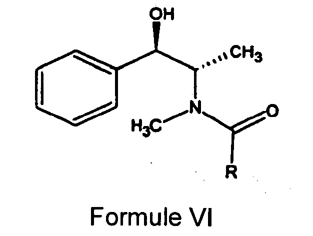 où R peut être choisi parmi un atome d'hydrogène, un groupe méthyle ou un groupe phényle ;
b) la désoxygénation des N-éphédrines acylées de formule VI pour donner un produit de formule VIII 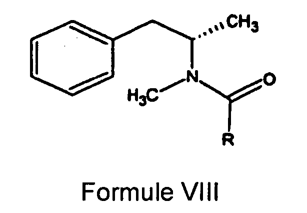 où R peut être tel que défini ci-dessus dans l'étape (a) ;
c) la réalisation d'une hydrolyse acide du composé de formule VIII pour obtenir le composé de formule II 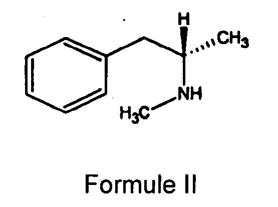
**caractérisé en ce que** la réaction de désoxygénation de l'étape (b) est réalisée en présence d'un catalyseur de nickel de Raney.

3. Procédé selon la revendication 1 ou 2, dans lequel l'agent acylation est choisi parmi le formate d'éthyle, le formate de méthyle, l'anhydride acétique et le chlorure de benzoyle.

4. Procédé selon la revendication 3, dans lequel l'agent d'acylation est le formate d'éthyle.

5. Procédé selon l'une quelconque des revendications précédentes, dans lequel le solvant utilisé pour la désoxygénation est choisi parmi le toluène ou le benzène.

6. Procédé selon la revendication 5, dans lequel le solvant utilisé pour la désoxygénation est le toluène.

7. Procédé selon l'une quelconque des revendications précédentes, dans lequel la température utilisée pour la réaction de désoxygénation est la température de reflux du solvant utilisé.

8. Procédé selon la revendication 7, dans lequel la température utilisée pour la réaction de désoxygénation est comprise dans la plage allant de 70°C à 85°C.

9. Procédé selon la revendication 8, dans lequel la température utilisée pour la réaction de désoxygénation est comprise dans la plage allant de 80°C à 85 °C.

10. Procédé selon l'une quelconque des revendications précédentes, **caractérisé en ce que** le rapport de N-acyl-éphédrine (g) au catalyseur de nickel de Raney (volume au repos, ml), pour la réaction de désoxygénation est de 1 g de substrat pour 6 ml de catalyseur.

11. Procédé selon la revendication 10, dans lequel le rapport de N-acyl-éphédrine (g) au catalyseur de nickel de Raney (volume au repos, ml), pour la réaction de désoxygénation est de 1 g de substrat pour 2 ml de catalyseur.

12. Procédé selon la revendication 1, 2 ou 3, **caractérisé en ce que** l'hydrolyse acide est réalisée en utilisant une solution aqueuse d'acide chlorhydrique.
